(19) Europäisches Patentamt
European Patent Office
Office européen des brevets

(11) Veröffentlichungsnummer: **0 271 790 B1**

(12) **EUROPÄISCHE PATENTSCHRIFT**

(45) Veröffentlichungstag der Patentschrift: **15.07.92**

(51) Int. Cl.⁵: **C07C 69/16**, C07C 69/63, C07C 67/29, C07C 67/287

(21) Anmeldenummer: **87118013.9**

(22) Anmeldetag: **05.12.87**

# (54) Verfahren zur Herstellung von phenoxysubstituierten Estern und Zwischenprodukte hierfür.

(30) Priorität: **13.12.86 DE 3642632**

(43) Veröffentlichungstag der Anmeldung:
**22.06.88 Patentblatt 88/25**

(45) Bekanntmachung des Hinweises auf die Patenterteilung:
**15.07.92 Patentblatt 92/29**

(84) Benannte Vertragsstaaten:
**AT BE CH DE ES FR GB IT LI NL SE**

(56) Entgegenhaltungen:
**EP-A- 0 040 350**
**EP-A- 0 236 884**

(73) Patentinhaber: **BASF Aktiengesellschaft**
**Carl-Bosch-Strasse 38**
**W-6700 Ludwigshafen(DE)**

(72) Erfinder: **Schulz, Guenter, Dr.**
**Carl-Bosch-Strasse 96**
**W-6700 Ludwigshafen(DE)**
Erfinder: **Sauter, Hubert, Dr.**
**Neckarpromenade 20**
**W-6800 Mannheim 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 271 790 B1

## Beschreibung

Die vorliegende Erfindung betrifft ein Verfahren zur Herstellung von phenoxysubstituierten Estern I,

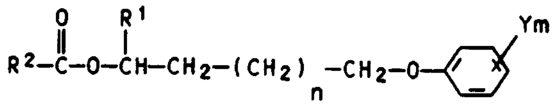

I,

in der

$R^1$ eine Gruppe

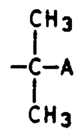

oder

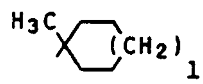

bedeutet, in der A $C_1$- bis $C_4$-Alkyl, Phenyl oder $C_7$- bis $C_{12}$-Aralkyl darstellt und l 0 oder 1 ist,

$R^2$ $C_1$- bis $C_4$-Alkyl,

n die Zahlen 0, 1, 2 oder 3,

m die Zahlen 1, 2 oder 3 bedeuten und

Y für Wasserstoff, Fluor, Chlor, Brom $C_1$- bis $C_3$-Alkyl, $C_1$- bis $C_3$-Alkoxy, Trifluormethyl, Phenyl oder Phenoxy steht, wobei, wenn m größer als 1 ist, die einzelnen Atome bzw. Gruppen Y gleich oder verschieden sind,

Zwischenprodukte hierfür und Verfahren zur Herstellung der Zwischenprodukte.

Die Verbindungen I sind wertvolle Ausgangsstoffe zur Herstellung von Pflanzenschutzmitteln, insbesondere zur Herstellung von Phenoxyalkanoltriazolverbindungen, z.B. das 8-(2-Fluorphenoxy)-4-(1,2,4-triazol-1-yl)-3-hydroxy-2,2-dimethyloctan wie aus der älteren Anmeldung P 36 06 947.7 (O.Z. 0050/38303) und dem EP 40 350 hervorgeht. Sie wurden bislang durch Umsetzung von Aldehyden, z.B. Pivalaldehyd, mit Grignardverbindungen, wie z.B. 4-(2-Fluorphenoxy)butylmagnesiumchlorid und Umsetzung der entstandenen Alkohole mit entsprechenden Säureanhydriden synthetisiert, was folgende Nachteile mit sich bringt: Zum einen geht ein großer Anteil der Grignardverbindung durch Hydridübertragung auf den sterisch gehinderten Aldehyd unter Bildung des terminalen Alkens verloren, zum anderen ist das der Grignardverbindung zugrundeliegende Halogenid schwer zugänglich.

Der Erfindung lag nun die Aufgabe zugrunde, einen einfachen und wirtschaftlichen Syntheseweg für die Verbindungen I zu finden.

Es wurde nun gefunden, daß sich die eingangs definierten Verbindungen I vorteilhaft herstellen lassen, indem man in einer ersten Stufe einen Diester der Formel II

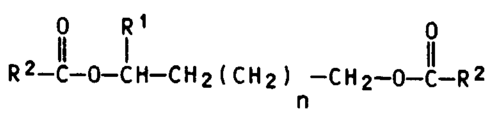

II

mit einem Äquivalent wäßrigen Alkali zum Alkohol der Formel IIIa

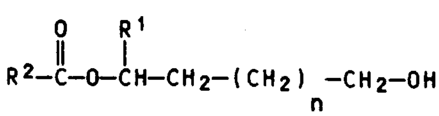

IIIa

verseift, diesen Alkohol in einer zweiten Stufe mit einem Halogenierungsmittel oder einem Sulfonylchlorid oder -bromid in eine Verbindung der Formel IIIb

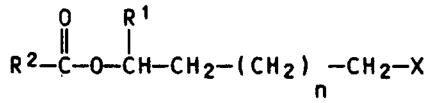

IIIb,

in der X für eine nukleophil verdrängbare Abgangsgruppe wie Chlor, Brom oder den Mesyl- oder Tosylrest steht, überführt und dann in einer dritten Stufe die Verbindung IIIb mit einem Phenolat der Formel IV

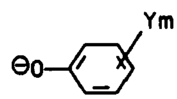

IV,

in der Y und m die vorgenannte Bedeutung haben in Gegenwart eines aprotischen Lösungsmittels zu Verbindung I umsetzt.

In den Verbindungen I, II und III steht der Rest $R^1$ für eine 1-Methylcyclohexyl- oder -cyclopentylgruppe oder die Gruppe $-C(CH_3)_2-A$, worin A $C_1$- bis $C_4$-Alkyl, z.B. Methyl-, Ethyl-, n-Propyl, Isopropyl oder n-Butyl, Phenyl oder einen $C_7$-$C_{12}$-Aralkylrest, z.B. Benzyl, Phenylethyl bedeutet. Der Rest $R^2$ stellt eine verzweigte oder vorzugsweise unverzweigte $C_1$- bis $C_4$-Alkylgruppe, z.B. Methyl, Ethyl, n-Propyl oder n-Butyl dar. Die Indizes n und m in Verbindung I bedeuten ganze Zahlen 1, 2 oder 3, n weiterhin 0. Bevorzugt sind Verbindungen I und II, in denen $R^1$ tert.-Butyl, $R^2$ Methyl- oder Ethyl und n die Zahl 3 darstellen.

Der Rest Y ist durch Wasserstoff, Fluor, Chlor, Brom, $C_1$- bis $C_3$-Alkyl oder -Alkoxy wie z.B. Methyl, Ethyl, Propyl, Iospropyl, Methoxy, Ethoxy, Propoxy und Isopropoxy gekennzeichnet.

In der ersten Stufe des Verfahrens wird der Diester II mittels einem Äquivalent wäßrigem Alkali, z.B. Natrium- oder Kaliumhydroxid oder auch andere Basen wie Erdalkalihydroxiden oder Carbonaten zum Alkohol IIIa verseift. Die Verseifung kann mit oder ohne Lösungsvermittler durchgeführt werden. Als Lösungsvermittler eignen sich z.B. aliphatische kurzkettige Alkohole wie Methanol, Ethanol, Propanol, Isopropanol oder Butanol. Die Reaktionstemperatur ist nicht besonders kritisch, sie liegt im allgemeinen bei 20 bis 100 °C. Da die Reaktion bei höherer Temperatur schneller abläuft als bei Raumtemperatur, sind Temperaturen von ca. 50 bis 80 °C bevorzugt.

Die so erhaltenen Alkohole werden dann in an sich bekannter Weise, z.B. durch Extraktion aus dem Reaktionsgemisch abgetrennt und nach vorheriger Reinigung oder vorzugsweise direkt mit einem Halogenierungsmittel oder Sulfonylchlorid oder -bromid in die Verbindung IIIb, in der X für eine nukleophil verdrängbare Abgangsgruppe, z.B. für den Mesyl- oder Tosylrest oder vorzugsweise für Chlor oder Brom steht, überführt.

Halogenierungsmittel zum Austausch der Hydroxlygruppe sind insbesondere anorganische Säurehalogenide wie Phosphorpentachlorid und vorzugsweise Phosphortrichlorid, Phosphortribromid, Thionylchlorid und Thionylbromid. Diese Umsetzung kann in Substanz oder in Gegenwart eines Verdünnungsmittels, z.B. eines inerten organischen Lösungsmittels wie Benzol, Toluol oder Xylol bei Temperaturen von 50 bis 100 °C vorgenommen werden. Typische Reaktionsbedingungen sind z.B. Houben-Weyl, Methoden der organischen Chemie, Band V/3, 1962, S. 862-864 und Band V/4, 1960, Seiten 389 ff zu entnehmen.

Die Überführung der Hydroxylgruppe in einen Sulfonsäureester mittels Sulfonylchlorid oder Sulfonylbromid wie para-Toluolsulfonsäurehalogenid oder Methansulfonsäurehalogenid kann in an sich bekannter Weise, z.B. wie in Houben-Weyl, Methoden der organischen Chemie, Band. V/4, 1960, S. 408 ff beschrieben in Substanz oder in Gegenwart von aprotischen Verdünnungsmittel wie Toluol oder Xylol ohne oder mit Säurefänger wie z.B. Pyridin, Chinolin oder Alkalicarbonat durchgeführt werden.

In einer dritten Stufe des Verfahrens wird die Verbindung IIIb, vorzugsweise nach destillativer Reinigung mit einem Phenolat IV in Gegenwart eines aprotischen Lösungsmittels wie Tetramethylharnstoff, N,N-Dimethylethylen- oder -propylenharnstoff, Dimethylsulfoxid und insbesondere Dimethylformamid, N-Methylpyrrolidon oder Xylol umgesetzt.

Das Phenolat kann in situ aus dem entsprechenden Phenol mittels einer geeigneten Base, die die Phenole deprotonieren kann, z.B. Alkalihydroxid wie Natrium- oder Kaliumhydroxid hergestellt werden. Es ist selbstverständlich auch möglich, das Phenolat getrennt aus dem Phenol und Basen wie Alkalihydroxiden oder $C_1$- bis $C_4$-Alkoholaten wie Methylat oder Ethylat herzustellen und dann mit den Verbindungen IIIb umzusetzen. Die Phenolate enthalten 1 bis 3 gleiche oder verschiedene Substituenten Y, die wie oben angegeben, definiert sind. $C_1$- bis $C_3$-Alkyl- oder Alkoxyreste sind beispielsweise Methyl, Ethyl, Propyl, Isopropyl, Methoxy, Ethoxy, Propoxy oder Isopropoxy.

Eine besonders vorteilhafte Ausführungsform des Verfahrens sei am Beispiel des Diacetats

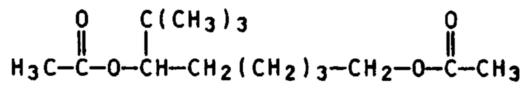

aufgezeigt. Dieses kann durch Verrühren mit 1 Äquivalent 50 %iger Natronlauge bei 60 bis 80° zum Monoacetat

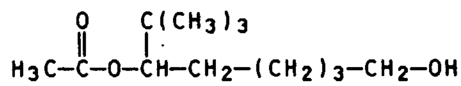

verseift und nach Abtrennung aus dem Reaktionsgemisch mit 1 bis 2 mol Thionylchlorid pro Mol Monoacetat bei Temperaturen von 60 bis 80° C in Xylol als Lösungsmittels in das Chlorid

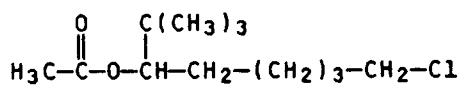

überführt werden. Nach Abtrennung von ggf. überschussigem Thionylchlorid aus dem Reaktionsgemisch oder vorteilhaft nach destillativer Reinigung dieses Zwischenprodukts kann die Umsetzung mit stöchiometrischen oder mehr als stöchiometrischen Mengen eines Phenolates der allgemeinen Formel IV, das man z.B. durch Deprotonierung des entsprechenden Phenols mit Alkalihydroxid erhalten hat, in Xylol oder vorzugsweise Dimethylformamid als Lösungsmittel bei Temperaturen von 60 bis 120° C zur gewünschten Phenoxyverbindung vorgenommen werden.

Den für die beschriebene Reaktionssequenz benötigten Diester der Formel II

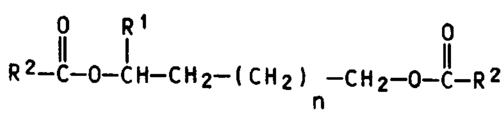

II

in der $R^1$, $R^2$ und n die in Anspruch 1 genannte Bedeutung haben, erhält man, indem man ein Lacton der Formel V

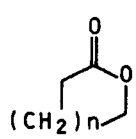

V

mit einer Grignard-Verbindung VI

$R^1MgZ$ VI,

worin Z Chlor oder Brom bedeutet, in einem unpolaren organischen Lösungsmittel umsetzt, wobei pro Mol Lacton 2 bis 3 mol der Grignard-Verbindung verwendet werden und man das Reaktionsgemisch anschließend mit 2 bis 3 mol Säureanhydrid VII

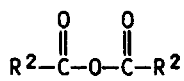

VII

je Mol Lacton behandelt.

Das gute Gelingen dieses Verfahrens ist überraschend, da bekannt ist, daß man Grignard-Verbindungen mit Aldehyden und Ketonen zwar in guten Ausbeuten zu Carbinolen umsetzen kann, daß aber die Umsetzung mit Säurederivaten wie Säurechloriden, Säureanhydriden und Estern im allgemeinen nicht zu einheitlichen Produkten führt (vgl. Jerry March, Advanced Organic Chemistry, 2nd Ed., Mc Graw-Hill 1977, S. 439 und 440 und dort zitierte Literatur). In der Regel erhält man Produktgemische, die z.B. durch zweimalige Grignard-Addition entstehen. Desweiteren ist die Folgereaktion zum Teil gebildeter Produkte mit noch unumgesetzten Ausgangsstoffen möglich. Um eine glatte Reaktion mit Säurederivaten sicherzustellen, muß man entweder Katalysatoren zusetzen oder anstelle der Grignard-Verbindungen auf andere metallorganische Verbindungen ausweichen.

In den für das erfindungsgemäße Verfahren verwendeten Grignard-Verbindungen steht $R^1$ für den in Anspruch 1 definierten Rest $R^1$, vorzugsweise für eine tertiäre Alkyl-, insbesondere tert.-Butylgruppe. Aus Kostengründen werden Alkylmagnesiumchloride bevorzugt verwendet.

Lactone V sind beispielsweise $\gamma$, $\delta$ oder $\epsilon$-Lactone wie $\gamma$-Butyrolacton, $\delta$-Valerolacton und $\delta$-Caprolacton.

Die Herstellung der Grignard-Verbindung kann zweckmäßig in einem inerten Lösungsmittel wie Ethern, z.B. Diethylether oder Tetrahydrofuran aus dem Chlorid $R^1Cl$ oder Bromid $R^1Br$ und Magnesium in an sich bekannter Weise vorgenommen werden. Die im Ether, vorzugsweise in 1 bis 5 mol je Mol Grignard-Verbindung gelöste Grignard-Verbindung wird direkt oder nach Entfernen von gegebenenfalls überschüssigem Ether mit dem Lacton umgesetzt, wobei als Lösungs- bzw. Verdünnungsmittel für diese Reaktion unpolare organische Verbindungen wie z.B. Alkane mit 4 bis 8 Kohlenstoffatomen oder vorteilhaft Aromaten wie Benzol, Toluol oder insbesondere Xylol eingesetzt werden können. Die Menge des Lösungs- bzw. Verdünnungsmittels sollte vorteilhaft so bemessen werden, daß das Reaktionsgemisch gut rührbar ist und die bei der exothermen Reaktion entstehende Wärme noch gut abgeführt werden kann. Anhand von Vorversuchen kann die optimale Lösungsmittelmenge für jede Reaktion leicht ermittelt werden. Im Fall von Xylol als Lösungsmittel haben sich Mengen von ca. 1 Liter pro Mol Grignard-Verbindung bewährt.

Pro Mol Lacton werden 2 bis 3 mol der Grignard-Verbindung umgesetzt. Die Reaktionstemperatur kann im allgemeinen bei ca. 20 bis 100, insbesondere 40 bis 90 ° C liegen.

Anschließend wird das Reaktionsgemisch mit einem Säureanhydrid IV, z.B. dem Anhydrid der Essigsäure, Propionsäure, Buttersäure oder Valeriansäure bei Temperaturen von 20 bis 100 ° C, insbesondere 30 bis 60 ° C umgesetzt, wobei pro Mol Lacton 2 bis 3 mol Anhydrid zugesetzt werden.

Nach an sich üblicher Aufarbeitung des Reaktionsgemisches z.B. durch Zugabe von Wasser, Phasentrennung und Abtrennung des Lösungsmittels fällt der gewünschte Diester II in der Regel in ausreichender Reinheit an, so daß er direkt zur nachfolgenden Verseifung eingesetzt werden kann. Natürlich ist auch vorab eine destillative Reinigung möglich.

Im Hinblick auf die Weiterverarbeitung der Zwischenprodukte II zu Pflanzenschutzmitteln wie z.B. in der älteren Anmeldung P 36 06 947.7 beschrieben, ist das erfindungsgemäße Verfahren besonders interessant zur Herstellung von Verbindungen, in denen $R^1$ eine tert.-Butylgruppe, $R^2$ eine Methyl- oder Ethylgruppe und n die Zahl 3 bedeutet.

Am Beispiel der Herstellung des Acetats von 8-(2-Fluorphenoxy)-2,2-dimethyloctan-3-ol werden die einzelnen Verfahrensschritte nachfolgend exemplarisch dargestellt.

Beispiel

1) Umsetzung von Caprolacton mit tert.-Butylmagnesiumchlorid und Acetanhydrid zum Diacetat

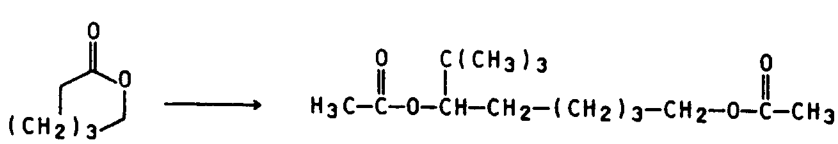

A B

In 2,89 mol tert. Butylgrignard mit 2,89 mol Tetrahydrofuran in Xylol, ergebend ein Gesamtvolumen von 3 l, werden bei 40 ° C Anfangstemperatur unter kräftigem Rühren 1,31 mol Caprolacton A in 100 ml Xylol innerhalb von ca. 2 min eingetragen. Die stark exotherme Reaktion führt bei Wärmeabführung durch Eis/Wasserkühlung und Lösungsmittelrückfluß zu einer Endtemperatur von 80 - 90 ° C. Man rührt 1 h nach und setzt bei ca. 30 ° C 2,89 mol Acetanhydrid so zu, daß eine Temperatur von ca. 60 ° C nicht wesentlich überschritten wird. Nach etwa 1 h werden 1,5 l Wasser zugesetzt und die Phasen getrennt. Eindampfen der organischen Phase führt zu einer Rohausbeute von 319,3 g, was bei einer Reinheit von ca. 64 % nach GC-Analyse (GC = Gaschromatographie) einer Diacetatausbeute von B von 60 % entspricht. Eine entsprechende Menge kann destillativ isoliert werden ($Kp_{18}$: 163 ° C). Das Rohprodukt kann jedoch auch direkt zur Verseifung eingesetzt werden.
$^1$H-NMR ($CDCl_3$): 1,9 s (9H), 1,2 - 1,75 m (8H), 2,02 s (3H), 2,05 s (3H), 4,05 t (2H), 4,72 dd (1H).

2. Verseifung des Diacetats B und Halogenierung des Monoacetats

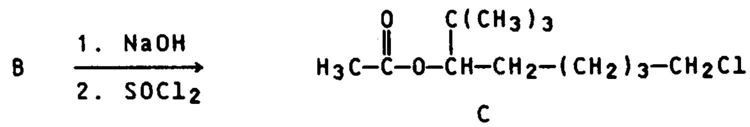

C

a) Verseifung:

Zu 0,81 mol Diacetat B werden bei 65 - 75 ° C unter kräftigem Rühren innerhalb von 60 min 0,81 mol 50 %ige Natronlauge getropft. Bei ca. 95 % GC-Ausbeute an Monoacetat wird abgebrochen. Man trennt die Phasen und extrahiert die wäßrige Phase 2 mal mit Xylol. Zur Trocknung wird die mit den Xylolextrakten vereingte Monoacetatphase nach Vortrocknen mit $Na_2S0_4$ andestilliert.

b) Chlorierung:

Man setzt soviel Lösungsmittel zu, daß eine ca. 30 %ige Lösung von Monoacetat in Xylol entsteht. Bei ca. 80 ° C werden etwa 1,5 Äquivalente Thionylchlorid zugetropft. Nach Ende der Gasentwicklung werden im Vakuum zunächst leichter siedende Anteile entfernt. Nach Entsäuerung mit Hydrogencarbonat wird im Vakuum destilliert. Man erhält 161 g (0,68 mol) des Produktes C ($Kp_2$: 145 - 150 ° C)
$^1$H-NMR ($CDCl_3$): 1,9 s (9H), 1,25 - 1,8 m (8H), 2,1 s (3H), 3,62 t (2H), 4,75 m (1H)

3. Umsetzung des Chlormonoacetats C zur phenoxysubstituierten Verbindung D

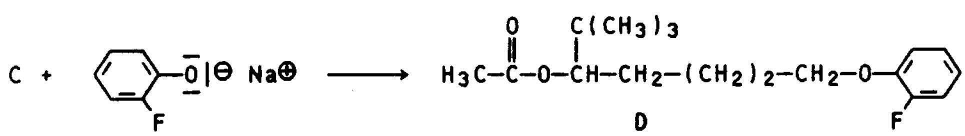

D

0,95 mol Natrium-2-Fluorphenolat in 300 ml trockenem DMF werden bei 70 ° C mit 0,95 mol C tropfenweise versetzt. Man rührt bei 80 ° C nach und verfolgt die Reaktion dünnschichtchromatographisch. Der Endpunkt wird durch den vollständigen Verbrauch des Phenolats angezeigt. Man filtriert und destilliert Dimethylformamid (DMF) weitgehend im Vakuum ab. DMF-Reste werden durch Aufnehmen in Methylenchlorid und Extraktion mit Wasser entfernt. Nach Entfernung des Lösungsmittels im Vakuum erhält man 265 g (0,85 mol) des Produktes D ($Kp_{0,1}$: 110 ° C).
$^1$H-NMR ($CDCl_3$): 1,9 s (9H), 1,2 - 1,9 m (8H), 2,08 s (3H), 4,0 t (2H), 4,75 dd (1H), 6,8 - 7,15 m (4H).

**Patentansprüche**

**1.** Verfahren zur Herstellung von Verbindungen der Formel I

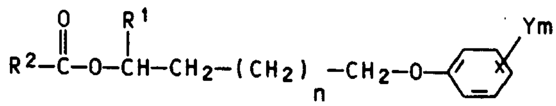
I,

in der

$R^1$ eine Gruppe

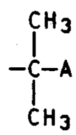
oder
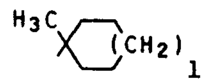

bedeutet, in der A $C_1$- bis $C_4$-Alkyl, Phenyl oder $C_7$- bis $C_{12}$-Aralkyl darstellt und l 0 oder 1 ist,

$R^2$ $C_1$- bis $C_4$-Alkyl,

n die Zahlen 0, 1, 2 oder 3,

m die Zahlen 1, 2 oder 3 bedeuten und

Y für Wasserstoff, Fluor, Chlor, Brom, $C_1$- bis $C_3$-Alkyl, $C_1$- bis $C_3$-Alkoxy, Trifluormethyl, Phenyl oder Phenoxy steht, wobei, wenn m größer als 1 ist, die einzelnen Atome bzw. Gruppen Y gleich oder verschieden sind,

dadurch gekennzeichnet, daß man in einer ersten Stufe einen Diester der Formel II

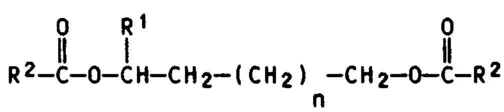
II

mit einem Äquivalent wäßrigem Alkali zum Alkohol der Formel IIIa

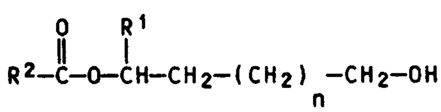
IIIa

verseift, diesen Alkohol in einer zweiten Stufe mit einem Halogenierungsmittel oder einem Sulfonylchlorid oder -bromid in eine Verbindung der Formel IIIb

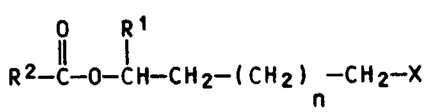
IIIb

in der X für eine nukleophil verdrängbare Abgangsgruppe wie Chlor, Brom oder den Mesyl- oder Tosylrest steht, überführt und dann in einer dritten Stufe die Verbindung IIIb mit einem Phenolat der Formel IV

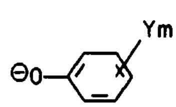
IV,

in der Y und m die vorgenannte Bedeutung haben, in Gegenwart eines aprotischen Lösungsmittels zu Verbindung I umsetzt.

**2.** Verfahren nach Anspruch 1, dadurch gekennzeichnet, daß $R^1$ tert.-Butyl, $R^2$ Methyl- oder Ethyl und n die Zahl 3 bedeuten.

**3.** Verfahren nach Anspruch 1, dadurch gekennzeichnet, daß man als Halogenierungsmittel in der zweiten Stufe ein Chlorierungs- oder Bromierungsmittel wie Thionylchlorid, -bromid, Phosphortribromid oder Phosphortrichlorid verwendet.

**4.** Verfahren nach Anspruch 1, dadurch gekennzeichnet, daß man das Phenolat in situ aus dem entsprechenden Phenol mittels einer Base erzeugt.

**5.** Verfahren nach Anspruch 1, dadurch gekennzeichnet, daß man als Lösungsmittel in der dritten Stufe Dimethylformamid, N-Methylpyrrolidon oder Xylol verwendet.

**6.** Verfahren zur Herstellung von Diestern der Formel II

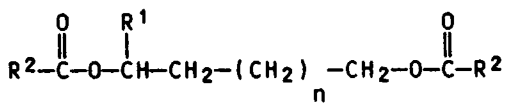

II,

in der $R^1$, $R^2$ und n die in Anspruch 1 genannte Bedeutung haben, dadurch gekennzeichnet, daß man ein Lacton der Formel V

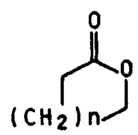

V

mit einer Grignard-Verbindung VI

$R^1MgZ$ VI,

worin Z Chlor oder Brom bedeutet, in einem unpolaren organischen Lösungsmittel umsetzt, wobei pro Mol Lacton 2 bis 3 mol der Grignard-Verbindung verwendet werden und man das Reaktionsgemisch anschließend mit 2 bis 3 mol Säureanhydrid VII

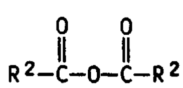

VII

je Mol Lacton behandelt.

**7.** Verfahren nach Anspruch 6, dadurch gekennzeichnet, daß man als Lösungsmittel $C_4$- bis $C_8$-Alkane, Benzol oder Alkylbenzol verwendet.

**8.** Verbindungen der Formel III

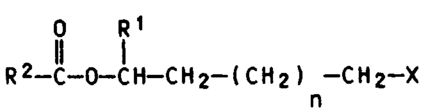

III,

in der

$R^1$ tert.-Butyl, tert.-Amyl, 1,1-Dimethylbenzyl oder die Gruppe

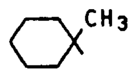

$R^2$ eine $C_1$- bis $C_4$-Alkylgruppe,
n die Zahlen 0, 1, 2 oder 3 und
X Hydroxi, Chlor, Brom, den Tosyl- oder Mesylrest

bedeuten.

**9.** Verbindungen gemäß Anspruch 8, in denen $R^1$ den tert.-Butylrest, $R^2$ einen Methyl- oder Ethylrest, n die Zahl 3 und X Hydroxi, Chlor oder Brom bedeuten.

**10.** Diester der Formel II

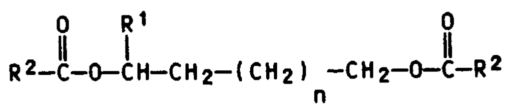

II.

in der $R^1$, $R^2$ und n die in Anspruch 8 genannte Bedeutung haben.

**11.** Diester gemäß Anspruch 10, in denen $R^1$ den tert.-Butylrest, $R^2$ einen Methyl- oder Ethylrest, und n die Zahl 3 bedeuten.

## Claims

**1.** A process for the preparation of a compound of the formula I

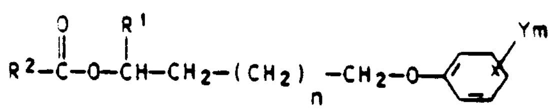

I

where $R^1$ is

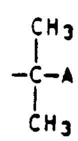

or

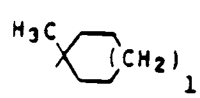

where A is $C_1$-$C_4$-alkyl, phenyl or $C_7$-$C_{12}$-aralkyl and 1 is 0 or 1, $R^2$ is $C_1$-$C_4$-alkyl, n is 0, 1, 2 or 3, m is 1, 2 or 3 and Y is hydrogen, fluorine, chlorine, bromine, $C_1$-$C_3$-alkyl, $C_1$-$C_3$-alkoxy, trifluoromethyl, phenyl or phenoxy, and, when m is greater than 1, the individual atoms or groups Y are identical or different, wherein, in a first stage, a diester of the formula II

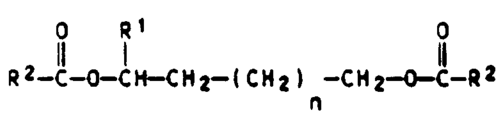

II

is hydrolyzed with one equivalent of an aqueous alkali to give the alcohol of the formula IIIa

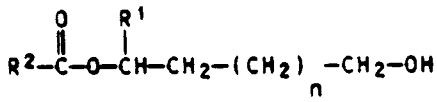

IIIa,

in a second stage this alcohol is converted with a halogenating agent or a sulfonyl chloride or bromide into a compound of the formula IIIb

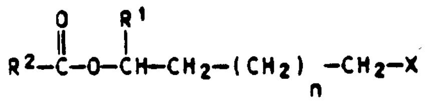

IIIb

where X is a nucleophilically displaceable leaving group, such as chlorine, bromine, mesyl or tosyl, and then, in a third stage, the compound IIIb is reacted with a phenolate of the formula IV

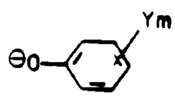

IV

where Y and m have the abovementioned meanings, in the presence of an aprotic solvent to give compound I.

**2.** A process as claimed in claim 1, wherein $R^1$ is tert-butyl, $R^2$ is methyl or ethyl and n is 3.

**3.** A process as claimed in claim 1, wherein a chlorinating or brominating agent, such as thionyl chloride or bromide, phosphorus tribromide or phosphorus trichloride, is used as the halogenating agent in the second stage.

**4.** A process as claimed in claim 1, wherein the phenolate is prepared in situ from the corresponding phenol by means of a base.

**5.** A process as claimed in claim 1, wherein dimethylformamide, N-methylpyrrolidone or xylene is used as the solvent in the third stage.

**6.** A process for the preparation of a diester of the formula II

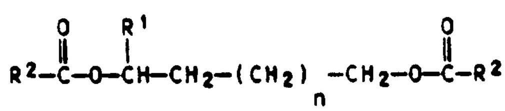

II

where $R^1$, $R^2$ and n have the meanings stated in claim 1, wherein a lactone of the formula V

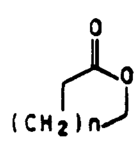

V

is reacted with a Grignard compound VI

$R^1MgZ$ VI

where Z is chlorine or bromine, in a nonpolar organic solvent, from 2 to 3 moles of the Grignard compound being used per mole of lactone, and the reaction mixture is then treated with from 2 to 3 moles of an anhydride VII

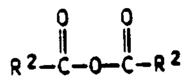

VII

per mole of lactone.

**7.** A process as claimed in claim 6, wherein the solvent used is a $C_4$-$C_8$-alkane, benzene or an alkylbenzene.

**8.** A compound of the formula III

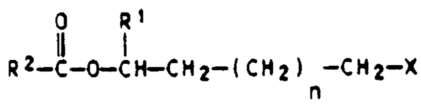

III

where $R^1$ is tert-butyl, tert-amyl, 1,1-dimethylbenzyl or the group

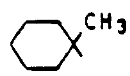

$R^2$ is $C_1$-$C_4$-alkyl, n is 0, 1, 2 or 3 and X is hydroxyl, chlorine, bromine, tosyl or mesyl.

**9.** A compound as claimed in claim 8, wherein $R^1$ is tert-butyl, $R^2$ is methyl or ethyl, n is 3 and X is hydroxyl, chlorine or bromine.

**10.** A diester of the formula II

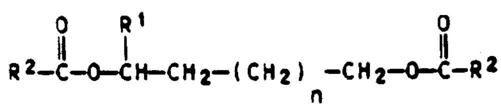

II

where $R^1$, $R^2$ and n have the meanings stated in claim 8.

**11.** A diester as claimed in claim 10, wherein $R^1$ is tert-butyl, $R^2$ is methyl or ethyl and n is 3.

## Revendications

**1.** Procédé de préparation de composés de formule I

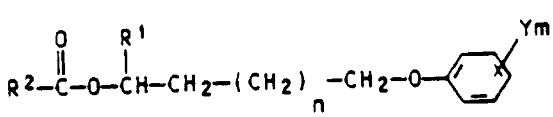

I.

dans laquelle

$R^1$ est un groupement

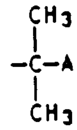 ou 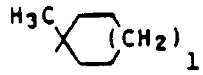

où A représente un reste alkyle en $C_1$ à $C_4$, phényle ou aralkyle en $C_7$ à $C_{12}$ et 1 est mis pour 1 ou 0,

$R^2$ est un radical alkyle en $C_1$ à $C_4$,

n est mis pour le nombre 0, 1, 2 ou 3,

m est mis pour le nombre 1, 2 ou 3 et

Y est mis pour un atome d'hydrogène, de fluor, de chlore, de brome, pour un radical alkyle en $C_1$ à $C_3$, alcoxy en $C_1$ à $C_3$, trifluorométhyle, phényle ou phénoxy, les atomes ou groupements Y individuels étant identiques ou différents lorsque m est supérieur à 1,

caractérisé en ce que, dans une première étape, on saponifie un diester de formule II

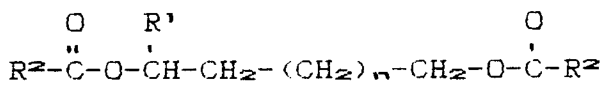 II

avec un équivalent d'une base aqueuse, pour obtenir l'alcool de formule IIIa

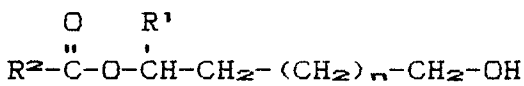 IIIa

on transforme cet alcool, dans une deuxième étape, avec un agent d'halogénation ou un chlorure ou bromure de sulfonyle, en un composé de formule IIIb

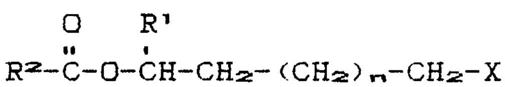 IIIb

dans laquelle X est mis pour un groupement séparable susceptible de substitution nucléophile, tel qu'un atome de chlore, de brome ou le reste mésyle ou tosyle, puis, dans une troisième étape, on fait réagir le composé IIIb avec un phénolate de formule IV

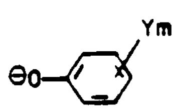 IV,

dans laquelle Y et m ont les significations données précédemment, en présence d'un solvant aprotique, pour obtenir le composé I.

**2.** Procédé selon la revendication 1, caractérisé en ce que $R^1$ représente un radical tert.-butyle, $R^2$ un radical méthyle ou éthyle et n le nombre 3.

**3.** Procédé selon la revendication 1, caractérisé en ce qu'on utilise, comme agent d'halogénation dans la deuxième étape, un agent de chloration ou de bromation tel que le chlorure ou le bromure de thionyle, le tribromure de phosphore ou le trichlorure de phosphore.

**4.** Procédé selon la revendication 1, caractérisé en ce qu'on produit le phénolate in situ à partir du phénol

correspondant au moyen d'une base.

**5.** Procédé selon la revendication 1, caractérisé en ce qu'on utilise, comme solvant dans la troisième étape, du diméthylformamide, de la N-méthylpyrrolidone ou du xylène.

**6.** Procédé de préparation de diesters de formule II

$$R^2-\overset{\overset{O}{\|}}{C}-O-\overset{\overset{R^1}{|}}{C}H-CH_2-(CH_2)_n-CH_2-O-\overset{\overset{O}{\|}}{C}-R^2 \qquad II$$

dans laquelle $R^1$, $R^2$ et n ont les significations données dans la revendication 1, caractérisé en ce qu'on fait réagir une lactone de formule V

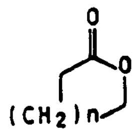

V

avec un composé de Grignard VI

$R^1MgZ$ VI

dans laquelle Z représente un atome de chlore ou de brome, dans un solvant organique non polaire, en utilisant, par mole de lactone, 2 à 3 moles du composé de Grignard, puis on traite le mélange réactionnel par 2 à 3 moles d'anhydride d'acide VII

$$R^2-\overset{\overset{O}{\|}}{C}-O-\overset{\overset{O}{\|}}{C}-R^2 \qquad VII$$

par mole de lactone.

**7.** Procédé selon la revendication 6, caractérisé en ce qu'on utilise, comme solvant, les alcanes en $C_2$ à $C_8$, le benzène ou un alkylbenzène.

**8.** Composés de formule

$$R^2-\overset{\overset{O}{\|}}{C}-O-\overset{\overset{R^1}{|}}{C}H-CH_2-(CH_2)_n-CH_2-X \qquad III$$

dans laquelle

- $R^1$ est un radical tert.-butyle, tert.-amyle, 1,1-diméthylbenzyle ou le groupement

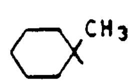

- $R^2$ est un groupement alkyle en $C_1$ à $C_4$,
- n est le nombre 0, 1, 2 ou 3 et
- X est un radical hydroxy, un atome de chlore, de brome ou le reste tosyle ou mésyle.

**9.** Composés selon la revendication 8, dans lesquels $R^1$ représente le reste tert.-butyle, $R^2$ un reste méthyle ou éthyle, n le nombre 3 et X un radical hydroxy ou un atome de chlore ou de brome.

**10.** Diesters de formule II

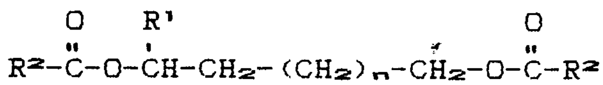

II

dans laquelle $R^1$, $R^2$ et n ont les significations données dans la revendication 8.

**11.** Diesters selon la revendication 10, dans lesquels $R^1$ représente le reste tert.-butyle, $R^2$ un reste méthyle ou éthyle et n le nombre 3.